Europäisches Patentamt

(19) European Patent Office

Office européen des brevets

(11) Publication number: **0 088 108**
**B1**

## EUROPEAN PATENT SPECIFICATION

(45) Date of publication of patent specification: **29.04.87**

(21) Application number: **82902764.8**

(22) Date of filing: **30.07.82**

(86) International application number:
**PCT/US82/01041**

(87) International publication number:
**WO 83/00437 17.02.83 Gazette 83/05**

(51) Int. Cl.⁴: **A 61 K 39/112, C 12 N 1/20,
C 12 N 15/00**

(54) **ORAL VACCINE FOR IMMUNIZATION AGAINST ENTERIC DISEASE.**

(30) Priority: **31.07.81 US 289013**

(43) Date of publication of application:
**14.09.83 Bulletin 83/37**

(45) Publication of the grant of the patent:
**29.04.87 Bulletin 87/18**

(84) Designated Contracting States:
**AT BE CH DE FR GB LI NL SE**

(56) References cited:
**EP-A-0 080 806**
**US-A-3 856 935**

**Infection and Immunity, Volume 24, No.1,
issued 1979 (United States), Diena et al.,
"Mouse Protective Capabilites of Escherichia
coli Hybridis Expressing Salmonella typhi
Antigens", See pages 90-93**

**Infectious Diseases, Volume 145, No. 3, issued
1982 (United States), Wahdam et al., "A
Controlled Field Trial of Live Salmonella typhi
Strain Ty 21a Oral Vaccine Against Typhoid:
Three-Year Results", See pages 292-295**

(73) Proprietor: **Swiss Serum and Vaccine Institute
Berne
Rehhagstrasse 79
CH-3001 Berne (CH)**

(72) Inventor: **Formal, Samuel B.
11405 Woodson Avenue
Kensington, MD 20895 (US)**
Inventor: **Baron, Louis S.
713 Horten Drive
Silver Spring, MD 20902 (US)**
Inventor: **Kopecko, Dennis J.
4601 Flower Valley Drive
Rockville, MD 20853 (US)**

(74) Representative: **Bannerman, David Gardner
et al
Withers & Rogers 4 Dyer's Buildings Holborn
London, EC1N 2JT (GB)**

(56) References cited:
**Infection and Immunity, Volume 29, No. 1,
issued 1980 (United States), Kopecko et al.,
"Genetic and Physical Evidence for Plasmid
Control of Shigella Sonnei Form I Cell Surface
Antigen", See pages 207-214**

Courier Press, Leamington Spa, England.

(56) References cited:

**CHEMICAL ABSTRACTS, vol. 95, no. 19, November 9, 1981, page 402, abstract no. 165464d COLUMBUS OHIO (US) D.J. KOPECKO et al.: "Invasive bacterial pathogens of the intestine: Shigelle virulence plasmids and potential vaccine approaches".**

**INFECTION AND IMMUNITY, vol. 37, no. 1, July 1982 D.F. KEREN et al.: "Intestinal Immunoglobulin A Responses in Rabbits to a Salmonella typhi Strain Harboring a Shigella sonnei Plasmid", page 387-389.**

**INFECTION AND IMMUNITY, vol. 34, no. 3, December 1981 S.B. FORMAL et al.: "Construction of a Potential Bivalent Vaccine Strain: Introduction of Shigella sonnei Form I Antigen Genes into the ga1E Salmonella typhi Ty21a Typhoid Vaccine Strain", pages 746-750.**

**ABSTRACT BOOKLET page 2, handed out by Dr. KOPECKO to participants of the Proc. Int. Plasmid Conf., 1981 (see page 59 of the file)**

## Description

Background of the invention

This invention relates broadly to a class of oral vaccines for the prevention of enteric disease. A living, non-pathogenic mutant, oral vaccine strain of *Salmonella typhi* has already been shown to be safe and effective in protecting against typhoid fever; it is a mutant, galactose epimeraseless strain of *S. typhi* designated as Ty21a. Its preparation, safety, and efficacy as an oral vaccine have already been described in Germanier, R. and E. Furer, J. Infect. Dis. *131*:553—558, 1975; Wahdan, N. H. et al., Bull. WHO. *58*:469—474; and U.S.—A—3,856,935 to R. Germanier.

Bacterial diseases of the gastrointestinal tract usually occur by one of three overall mechanisms. The first mechanism, termed "intoxication," occurs by bacterial secretion of an exotoxin that oftentimes is preformed in food prior to ingestion by the host. This process is exemplified by staphylococcal or clostridial food poisoning. In contrast, the remaining two processes require living and multiplying disease agents. In the "enterotoxigenic" mechanism, bacteria colonize the small intestine, usually in the jejunum or duodenum. These bacteria multiply on the intestinal surface and elaborate an enterotoxin that stimulates excessive fluid and electrolyte efflux resulting in a watery diarrhea. Enterotoxigenic *Escherichia coli* and *Vibrio cholera* serve as typical examples. Finally, a third group of organisms, termed "invasive", actually penetrate the epithelial mucosa of the large intestine. Subsequently, these organisms multiply intracellularly and disseminate within or through the mucosa. This latter mechanism, classically typified by *Shigella* and *Salmonella,* is now thought to be used by invasive strains of *E. coli, Yersinia,* and, possibly, *Campylobacter.* In contrast to other invasive bacterial diseases like salmonellosis, in which the invading bacteria are disseminated throughout the host, shigellosis is a disease normally confined to the intestinal lining. Thus, these features distinguish the toxigenic from the invasive mechanism of intestinal disease.

Two common and essential features of invasive bacteria are their ability to penetrate and to multiply within the epithelial cells of the colon. Mutants of *Shigella* strains that fail to penetrate or that penetrate but cannot multiply intracellularly have been isolated. Both types of mutants are avirulent. The process of invasion has thus far been characterized in microscopic, but not biochemical detail. The first visible alteration in the host intestinal epithelium is a localized destruction of the microvilli, the outermost structure of the intestinal lining. The invading bacteria are then engulfed by means of an invagination of the intestinal cell membrane and are contained intracellularly within vacuoles. Subsequently, the microvilli are reestablished and intracellular bacterial multiplication occurs. These bacterial then destroy the vacuole and disseminate to adjacent cells, causing necrosis and resulting in acute inflammation and focal ulceration of the epithelium. The resulting dysentery is characterized by a painful bloody, and mucous diarrhea, normally of relatively small volume.

Genetic studies of *Shegella flexneri* have previously resulted in the conclusion that virulence is multideterminant, with at least two widely separated bacterial chromosomal regions being required for invasion. Furthermore, these studies have shown that not only is a smooth lipopolysaccharide bacterial cell surface necessary for intestinal invasion, but also that only certain O-repeat unit polymers are effective in this process; this is true for both shigellae and invasive *E. Coli.* Until recently, plasmids did not appear to play a role in the invasion process or in the virulence of *Shigella.* Recent evidence amassed over the past three years, however, demonstrates that plasmids of *Shigella* are involved in the invasion process.

Bacillary dysentery remains highly endemic in many areas of the world and still is a significant cause of illness in developed countries. There are over thirty serotypes of the organisms which cause shigellosis (bacillary dysentery), the prominent members of which are *Shigella sonnei, Shigella flexneri, Shigella dysenteriae* and *Shigella bovdii.* There are six serologically separable *Shigella flexneri* types (i.e. I through IV) of which *Shigella flexneri* types IIa and III are responsible for the majority of *Shigella flexneri* infections. In the United States and northern Europe, *Shigella sonnei* is responsible for more than 65 percent of the cases. Together with *Shigella flexneri* IIa and III strains, *Shigella sonnei* strains cause greater than 90% of all shigellosis worldwide. Parenteral vaccines have not been effective in protecting against bacillary dysentery because shigellosis is an infection limited to the superficial layer of the colonic mucosa. It is, therefore, not surprising that attempts to immunize man or other primates with killed vaccines or even living virulent organisms, administered by the parenteral route, have not been successful.

Living, attenuated, oral *Shigella* strain vaccines have been demonstrated to be protective against bacillary dysentery under both laboratory and field conditions. It has been suggested that the local intestinal immune response which is induced by the living oral vaccines inhibits invasion of intestinal epithelial cells by the pathogen. This immunity has been associated with the type-specific somatic antigen of the vaccine strain. None have come into widespread use because of difficulties in isolating safe, genetically-stable (non-reverting) strains or because of the large number of doses required to produce immunity.

*Shigella sonnei* produce a characteristic cell surface antigen, termed form I, which has altruonic acid as a component of its O-specific side chain. Recently, it has been demonstrated that the form I antigen is encoded by a large non-conjugative plasmid (Kopecko, D. J. et al., Infect. Immun. *29*:207—214, 1980). This fundamental study found that by utilizing a plasmid mobilizing system, transfer of the form I antigen synthesizing genes was possible to certain specific *Shigella flexneri* and *Salmonella typhi* strains, or retransfer to a form II *Shigella sonnei* strain was possible.

Unlike shigellae, the typhoid bacillus causes a systemic infection following penetration of the intestinal mucosa, and parenteral vaccines have been shown to be effective against this infection. These parenteral vaccines do, however, elicit significant side effects which include fever, malaise, headache, and localized reactions at the site of inoculation. Since the safety (i.e., freedom from the above-mentioned side effects) and Immunogenicity of *Salmonella typhi* strain Ty21a has been established, it was considered that this attenuated strain might be utilized as a carrier organism for other protective antigens and, thus, could be used as an oral vaccine to protect against an enteric infection other than typhoid fever or simultaneously against both typhoid fever and also other enteric infections. By protective antigen we mean a molecular structure, either somatic or soluble, which stimulates production of one or more antibodies and protects against a specific enteric disease. Additionally, the term "carries", "carried" or "carrier" is not to be construed as limiting the invention to a specific derivative or method of modification of the parent *galE Salmonella typhi* strain. The term does not imply that the genetic determinant necessarily has to be plasmid-borne; conversely, it can be part of the bacterial chromosome.

Description of the invention

This invention is directed to a living, attenuated, oral vaccine capable of immunizing against only a single (non-typhoid) enteric disease or simultaneously against more than one enteric disease. It is recognized that shigellosis can be caused by any one of four distinct species and in the context of this invention may be considered four separate enteric diseases. The invention is based on an attenuated galactose epimeraseless strain of *Salmonella typhi*, proven to be safe for prevention of typhoid fever, which has been genetically engineered to carry the genetic determinants of additional protective antigens. In addition to typhoid fever, enteric infections caused by other organisms are considered amenable to treatment with a vaccine according to this invention; e.g., these organisms include various *Shigella* strains such as *Shigella sonnei, Shigella flexneri, Shigella dysenteriae, Shigella boydii*.

A particular vaccine of interest according to this invention is a living, attenuated, oral vaccine to be used to protect against both typhoid fever and bacillary dysentery due to *Shigella sonnei*. As mentioned above, the synthesis of the form I protective antigen of *Shigella sonnei* is encoded for on a large non-conjugative plasmid (Kopecko, D. J. et al., Infect. Immun. *29*:207—214, 1980). This plasmid was transferred to a streptomycin-resistant mutant of the attenuated *S. typhi* Ty21a vaccine strain. The resulting transconjugant strain 5076-1-C (on deposit at the American Type Culture Collection, Rockville, Maryland— ATCC No. 31904) expresses the somatic antigens of both *Salmonella typhi* and *Shigella sonnei* form I. The transconjugant strain produced antibodies in rabbits to both *Salmonella typhi* and *Shigella sonnei* and this strain also protected mice against intraperitoneal challenge with either virulent *Salmonella typhi* or *Shigella sonnei* cells. Strain 5076-1-C is considered to be a candidate, attenuated, oral vaccine strain which is intended to protect against both clinical typhoid fever and bacillary dysentery due to *Shigella sonnei*.

Construction of the donor strain

The F'$_{ts}$*lac*::Tn3 plasmid was introduced into *Shigella sonnei* I strain 53G by mating this strain with *E. coli* strain HU 679. The mating was incubated at 32°C and clones were selected and purified on a minimal growth medium supplemented with lactose, nicotinic acid, aspartic acid, and 20 micrograms per milliliter ampicillin. One resulting lactose-utilizing (Lac$^+$) and ampicillin resistant Ap$^R$clone was grown in Penassay broth (Difco) at 42°C to eliminate the F'*lac* plasmid. Sixteen Ap$^R$, Lac$^-$ form I 53G independent isolates were selected on MacConkey agar containing 20 μg/ml ampicillin. These 16 clones which now contain Tn*3* were remated with *E. coli* strain HU679, to regain the F'*lac*::Tn*3* plasmid. Stable Lac$^+$ *Shigella sonnei* form I clones, containing F'*lac*::Tn*3* from these matings were tested for their ability to transfer the form I antigen (along with the Lac$^+$-marker) to the recipient form II Shigella sonnei strain, which is *lac*$^-$, *met*$^-$, *nic*$^-$, *nal*$^R$. Conjugal mating mixtures were plated on minimal growth medium supplemented with lactose, nicotinic acid, aspartic acid, methionine, and 50 μg/ml nalidixic acid. One donor strain (5006-7-3) was observed to transfer form I-synthesizing ability. One resulting clone (5022-1C-9) was then used as a donor organism to transfer the form I antigen to *Shigella flexneri* IIa strain M42-43. Mating mixtures of strains 5022-1C-9 and M42-43 were plated on minimal growth medium supplemented with lactose, nicotinic acid and aspartic acid. A resulting isolate of *Shigella flexneri,* strain 5054-6-1, which inherited the form I antigen was employed as the donor strain to transfer the form I antigen to *Salmonella typhi* strain Ty21a.

Recipient strain

*Salmonella typhi* strain Ty21a which is commercially available under the mark VIVOTIF was obtained by L. S. Baron from R. Germanier of the Swiss Serum Institute, Berne, Switzerland, the assignee of U.S. Patent No. 3,856,935 to Germanier. For experimental purposes, a streptomycin resistant mutant of this strain was obtained by mutagenesis. The desirable carrier characteristics of the parent galactose epimeraseless mutant *Salmonella typhi* Ty21a strain were retained while allowing for experimental selection.

Construction of the *S. typhi-S. sonnei* I transconjugant strain 5076-1C

Donor strain 5054-6-1 *Shigella flexneri* harbouring the *Shigella sonnei* form I plasmid) was mated with the streptomycin resistant mutant of *Salmonella typhi* strain by Ty21a. Approximately 1×10$^9$ donor and

$1 \times 10^{10}$ recipient cells were mixed on the surface of a minimal agar growth medium supplemented with lactose, tryptophan, cystine, casamino acids and streptomycin.

Lac$^+$ *Salmonella typhi* transconjugants were selected on an appropriate minimal agar growth medium. The resulting Lac$^+$ clones were repurified by restreaking twice on identical medium and, then, were examined serologically. One Lac$^+$ isolate, designated strain 5076-1C, which was agglutinated by both *Salmonella typhi* and *Shigella sonnei* from 1 antisera, was selected for further study. This strain was found to have the serological characteristics of the recipient *Salmonella typhi* Ty21a strain and also expressed the *Shigella sonnei* form I antigen.

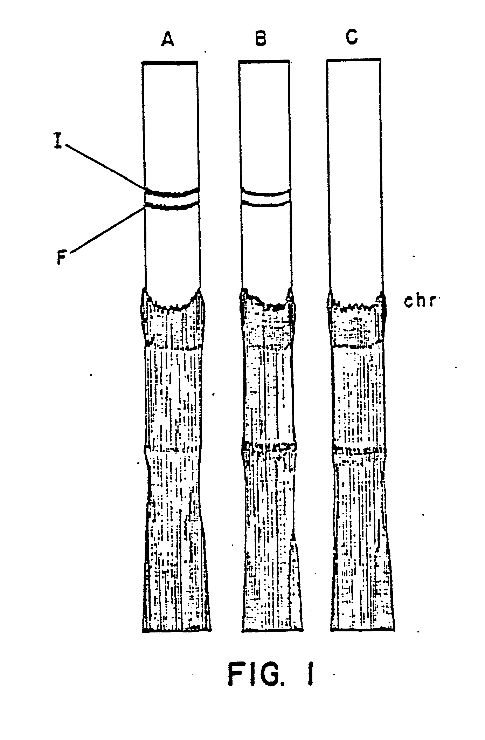

The *Salmonella typhi* transconjugant 5076-1C was expected to have received both the F'$_{ts}$*lac*::Tn*3* mobilizing plasmid and the form I plasmid. To demonstrate the presence of these plasmids in strain 5076-1C, plasmid DNA was prepared from the donor, recipient, and transconjugant strains. A comparison of the plasmid content of the parental and transconjugant strains was made by examination of their plasmid profiles following agarose gel electrophoresis. Fig. 1 shows the agarose gel electrophoretic profiles of circular plasmid DNA obtained from the recipient *Salmonella typhi*-Ty21a, the donor *Shigella flexneri* 5054-6-1, and the transconjugant form I *Salmonella typhi* 5076-1C strains. This figure shows the plasmid profile of: (A) donor strain 5054-6-1; (B) transconjugant *Salmonella typhi* strain 5076-1C; and (C) recipient *Salmonella typhi* Ty21a strain. The gel position expected for fragmented or chromosomal DNA is indicated by "Chr". The positions of supercoiled molecules of the F'$_{ts}$*lac*::Tn*3* plasmid and the form I plasmid are indicated by an "F" and an "I", respectively. Some small plasmid DNA species can be seen below the chromosal band. The direction of electrophoresis is from top to bottom. As shown in Fig. 1, the recipient *Salmonella typhi* Ty21a strain contains no large plasmids. However, both the donor and transconjugant strains can be seen harboring two large plasmid species, which correspond to the independent F'$_{ts}$*lac*::Tn*3* (80 Mdal) and form I (120 Mdal) plasmids. These observations suggest that form I antigen synthesis in the *Salmonella typhi* transconjugant strain 5076-1C is due to the presence of the form I plasmid.

Serological characterization of form I-*galE S. typhi* derivative strain 5076-1C

As shown in Table 1, the *Salmonella typhi* transconjugant strain 5076-1C was agglutinated to high titer both by *Salmonella typhi* antisera and by *Shigella sonnei* form I specific antisera. Furthermore, an antiserum prepared against the *Salmonella typhi* galE, form I strain 5076-1C, was observed to agglutinate both *Salmonella typhi* and *Shigella sonnei* form I cells. As expected, *Salmonella typhi* Ty21a or 643W cells failed to agglutinate in *Shigella sonnei* form I antiserum and neither *Shigella sonnei* form I 53G nor the *Shigella flexneri*—form I donor strain, 5054-6-1, reacted in *Salmonella typhi* antiserum.

TABLE 1

Agglutinin and agglutinin adsorption studies with the parenteral and derivative *Salmonella typhi* and *Shigella sonnei* strains

| Antigen | Unabsorbed antisera | | | Antisera adsorbed with strain 5076-1C cells | |
| | *Salmonella typhi* 643W | *Shigella sonnei* I | 5076-1C | *Salmonella typhi* 643W | *Shigella sonnei* I |
| --- | --- | --- | --- | --- | --- |
| *Salmonella typhi* Ty21a | 2560* | <80 | 1600 | 160 | ND** |
| *Salmonella typhi* 643W | 2560 | <80 | 1600 | 160 | ND |
| *Shigella sonnei* 53G | <80 | 640 | 400 | ND | <80 |
| *Shigella flexneri* 5054-6-1 | <80 | 640 | 400 | ND | <80 |
| *Salmonella typhi*- form I 5076-1C | 2560 | 640 | 1600 | <80 | <80 |

*Reciprocal of the dilution in which agglutination was observed
**ND=Not done

In further studies, adsorption of *Shigella sonnei* form I specific antiserum cells of the *Salmonella*

5

*typhi*—form I strain 5076-1C reduced the titer against *Shigella sonnei* form I strain 53G to <1:80. Similarly, adsorption of *Salmella typhi* 643W antiserum with the form I *Salmonella typhi* 5076-1C cells lowered the titer against cells of *Salmonella typhi* 643W from 1:16000 to 1:160. Thus, the *Salmonella typhi galE,* form I strain 5076-1C produces both the normal *Salmonella typhi* somatic antigens as well as the *Shigella sonnei* form I antigen.

Galactose-induced sensitivity to Lysis

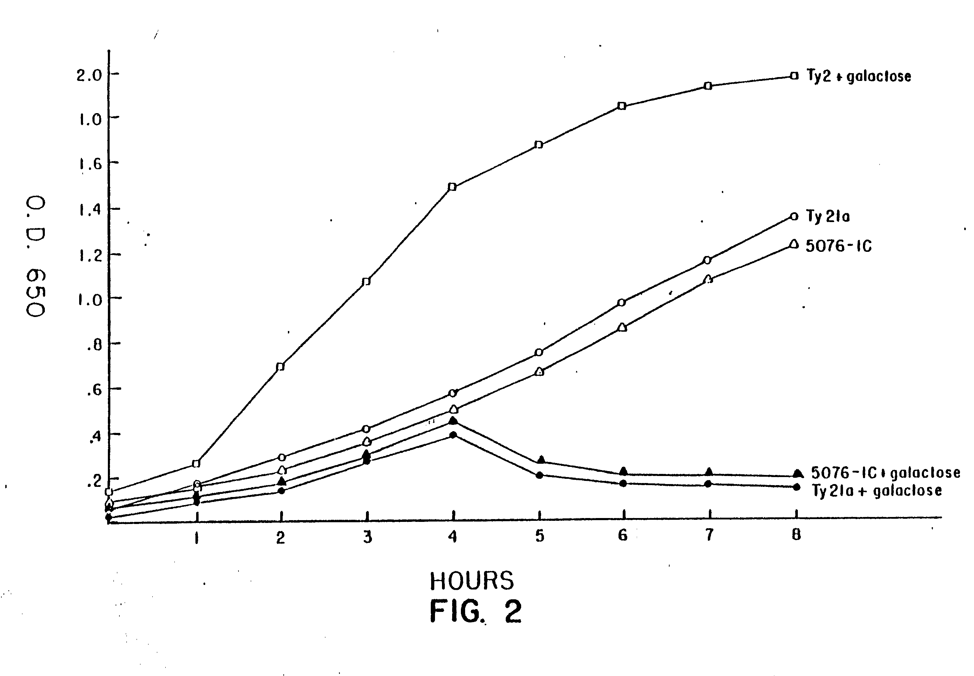

*Salmonella typhi* strain Ty21a contains a *galE* mutation that, following galactose uptake and the intracellular accumulation of galactose-1-phosphate and UDP-galactose, results in cell lysis. The growth patterns of *Salmonella typhi* strains Ty21a and 5076-1C, when grown in BHI broth in the presence or absence of 0.1% galactose, are shown in Fig. 2. Fig. 2 shows the sensitivity of *Salmonella typhi* strains Ty2, Ty21a and 5076-1C to growth in the presence or absence of galactose. The strains were grown in BHI with (closed symbols) or without (open symbols) 0.1% galactose. The control Gal$^+$ strain Ty2 grew equally well in both media. Both strains are similarly inhibited by the presence of galactose in the medium, relative to the control Gal$^+$ *Salmonella typhi* strain Ty2 which is not inhibited by galactose. Thus, strain 5076-1C behaves identical to the Ty21a strain under these conditions.

Persistence in mouse tissue of parenteral and transconjugant *galE Salmonella typhi* strains

Cells of *Salmonella typhi* Ty21a and the transconjugant form I strain 5076-1C were each injected IP into separate groups of 18 mice. Three animals were sacrificed at intervals of up to 15 days post-inoculation and the spleens were checked for the presence of bacteria. All animals harbored viable cells of either organism when the animals were sacrificed after 1 to 3 days post-inoculation. However, cultures of subsequently sacrificed animals were uniformly negative.

To test the safety of slightly larger doses of these vaccine strains, groups of 5 mice were injected IP with $1 \times 10^8$ cells of either *Salmonella typhi* Ty21a or the derivative strain 5076-1C, suspended in hog gastric mucin. All animals survived during the one week observation period. Therefore both the parenteral and transconjugant form I *galE Salmonella typhi* strains behaved as expected, i.e. viable cells do not persist in infected mice for longer than 3 to 6 days.

Mouse protection studies

Viable cells of *Salmonella typhi* Ty21a, *Shigella sonnei* 53G form I, and *Salmonella typhi galE,* form I strain 5076-1C, as well as the standard acetone-killed and dried (AKD) *Salmonella typhi* Ty2 cells were used to immunize mice, by either the IP or SC route. Control mice received inocula of saline. All mice were challenged 4 weeks post-immunization with either virulent *Salmonella typhi* or *Shigella sonnei* cells and deaths were recorded after 72 h. The results of these studies are summarized in Table 2. Each of the monovalent vaccines protected against homologous, but not against heterologous challenge. In contrast, the form I *Salmonella typhi* Ty21a derivative strain 5076-1C protected against challenge with either *Salmonella typhi* or *Shigella sonnei.*

TABLE 2

Protection of mice against *Salmonella typhi* and *Shigella sonnei* challenge with *Salmonella typhi* and *Shigella sonnei* vaccines

| Vaccine | Route of immunization | Chalienge strain* | |
|---|---|---|---|
| | | *Salmonella typhi* TY2 | *Shigella sonnei* 53GI |
| Living *Salmonella typhi* TY21a | IP | 0/12** | 15/15 |
| | SC | 0/15 | 15/15 |
| Living *Salmonella typhi*-form I 5076-1C | IP | 0/13 | 1/14 |
| | SC | 1/16 | 0/16 |
| Living *Shigella sonnei*-53GI | IP | 14/16 | 1/16 |
| | SC | 16/16 | 0/16 |
| AKD*** *Salmonella typhi* TY2 | IP | 2/16 | 15/16 |
| | SC | 1/16 | 16/16 |
| Saline | IP | 10/10 | 10/10 |

*Challenges, suspended in 0.5 percent hog gastric mucin, were administered IP.
**Deaths/Total recorded 72 hrs after challenge
***Standard acetone-killed and dried typhoid vaccine

6

Construction of additional galE *Salmonella typhi* genetic hybrid strains for use as oral vaccines

The general usefulness of the galE *Salmonella typhi* mutant as a protective antigen carrier for oral vaccine strain construction is further shown by the following experiments. *Shigella flexneri* serotype IIa possesses cell surface antigens that are coded for by the bacterial chromosome. Previous studies have revealed that the group antigenic determinants are closely linked to the *his* region, while the type antigenic determinants are located close to the *pro* region of the chromosome. An F'*lac* plasmid carrying the bacteriophage Mu *cts* 62 was introduced into *Shigella flexneri* IIa strain M4243 and the resultant strain, termed 6023-1-1, was maintained at 32°C, so as not to induce the Mu phage. Strain 6023-1-1 was conjugally mated at 37°C with an *Escherichia coli* strain that was *recA, mel, his, leu, met, arg, nal*[R] and a Mu *cts* 62 lysogen. Growth at 37°C would be expected to induce the Mu phage and cause transposition of the *his* region of the chromosome to the F'*lac* plasmid. Selection was made for an *E. coli* transconjugant that had received a F'*lac*-Mu plasmid that had picked up the *Shigella flexneri* IIa histidine genes and presumably the closely linked group antigenic genes. A His[+] transconjugant was isolated and this strain expressed the *Shigella flexneri* group (3, 4) cell surface antigens. This *E. coli* DK102 transconjugant strain was used as a donor to transfer the F'*lac*-Mu-*his* plasmid into a mutant of the *Salmonella typhi* TY21a strain. The *Salmonella typhi* TY21a strain was made Str[R] and His[-] (i.e. constructed by nitrosoguanidine mutagenesis and penicillin selection) and a Mu *cts* 62 lysogen. The resulting *Salmonella typhi* transconjugant strain carrying the F'*lac*-Mu-*his* plasmid was found to express both the *Salmonella typhi* 9, 12 antigens and the *Shigella flexneri* gp. 3, 4 antigens thus indicating that this *Salmonella typhi* hybrid strain (designated WR6003) would serve as an oral vaccine for immunization against disease caused by either *Salmonella typhi* or *Shigella flexneri* IIa organisms. A oral vaccine strain considered a better vaccine candidate against *Shigella flexneri* IIa than WR6003 has been constructed.

In experiments similar to those described immediately above again using the donor strain 6023-1-1, both the *his* and *pro* regions of the *Shigella flexneri* IIa chromosome were transferred, presumably via a recombinant F'*lac*-Mu-*his-pro* plasmid, into an intermediate *E. coli* strain, Mu *cts* 62 lysogen of AB1133 *pro, his, thr, leu, thi, arg*. This intermediate strain was then used to transfer the *Shigella flexneri his* and *pro* regions to a Mu *cts* 62 lysogenic, *his*[132], *str*[R] mutant of *Salmonella typhi* 9, 12 antigens and the *Shigella flexneri* type II and group (3, 4) antigens. This hybrid strain is considered an oral vaccine candidate that will protect against typhoid fever and shigellosis due to *Shigella flexneri* IIa. This hybrid oral vaccine strain, WR6000, has been deposited with the ATCC (ATCC No. 31931).

The various *Salmonella typhi* genetic hybrid oral vaccine strains whose construction has been described above (i.e., 5076-1C, WR6003, and WR6000) are considered exemplary of useful strains according to this invention. The 5076-1C *Salmonella typhi* hybrid strain carrying the *Shigella sonnei* form I plasmid is genetically unstable due to the natural instability of the form I plasmid. Although the 5076-1C strain is useful, the construction of more stable hybrid strains is desirable. It is important to emphasize that genetic hybrids of *Salmonella typhi* can be constructed by use of procedures, e.g., recombinant DNA procedures, other than standard classical genetic manipulation. Recombinant DNA techniques can be employed to make a desirable *Salmonella typhi* galE strain carrying the *Shigella sonnei* form I antigenic determinants. The form I genes of *Shigella sonnei* can simply be isolated and spliced, via known recombinant DNA techniques, into a small, genetically stable plasmid which can then be inserted into the galE *Salmonella typhi* strain. The resulting hybrid is considered desirable because of the stable form I antigen expression.

The galE *Salmonella typhi* genetic hybrid strains, discussed herein, are the active agents or components in preparation designed for use as oral vaccines according to this invention. These galE *Salmonella typhi* genetic hybrid cells can be dispersed in a pharmaceutical diluent such as a liquid suitable for ingestion by a human or lower animal host. Alternatively, the hybrid vaccine cells can be freeze-dried and administered in a solid form (e.g. as a tablet or capsule).

There may be physical limitations to the number of non-typhoid antigenic genes that can be introduced into a single galE *Salmonella typhi* strain. However, this invention provides for a multivalent vaccine, protective simultaneously against several diseases, which can be constructed by mixing several different *Salmonella typhi* genetic hybrid strains, each one producing different non-typhoid protective antigens. For example, according to this invention three different galE *Salmonella typhi* hybrid strains, each considered protective against typhoid fever and a different *Shigella* strain, can be constructed. One hybrid strain produces the *Shigella sonnei* form I antigen, a second hybrid strain produces the *Shigella flexneri* IIa somatic antigens and the third hybrid strain produces the *Shigella flexneri* III somatic antigens. Since *Shigella sonnei* and *Shigella flexneri* serotypes IIa and III are responsible for greater than ninety percent of all shigellosis worldwide, this vaccine is highly desirable.

The invention described herein is directed to a galE *Salmonella typhi* genetic hybrid strain, expressing at least one non-typhoid protective antigen, that can be used in an oral vaccine for immunization against enteric disease. The genetic manipulation of non-typhoid protective antigenic genes and their transfer to the galE *Salmonella typhi* carrier strain can be effected by a variety of procedures. The F'*lac*::T3 and F'$_{ts}$*lac*::Mu *cts*62 plasmids were employed in the construction of the genetic hybrid strains discussed above. However, similar genetic manipulations can be executed using other genetic systems. Also, any *Shigella sonnei* strain containing the form I plasmid can be used as a source of the form I antigenic genes and any *Shigella flexneri* IIa strain expressing type and group antigens can be used as a source of these antigenic genes.

The description of the subject invention includes detailed reference to specific embodiments to ensure a thorough understanding of the making and using thereof. It is to be understood, however, that these specific embodiments are considered merely exemplary of those within the scope of the invention defined by the claims which follow.

**Claims**

1. A living attenuated oral vaccine, for the immunization against enteric disease, comprising as the active component an effective dose of a genetic hybrid derivative of an attenuated galactose epimeraseless mutant strain of *Salmonella typhi* and a *Shigella* strain protective antigen carried thereby, or a mixture of said genetic hybrid derivatives.

2. An oral vaccine according to Claim 1 wherein the *Shigella* strain protective antigen is the form I antigen of *Shigella sonnei* and the genetic hybrid strain expresses both *Salmonella typhi* and *Shigella sonnei* antigens.

3. An oral vaccine according to Claim 2 wherein the form I antigen is plasmid encoded.

4. An oral vaccine according to Claim 1 wherein the *Shigella* strain protective antigen is a *Shigella flexneri* protective antigen.

5. An oral vaccine according to Claim 4 wherein the *Shigella* strain protective antigen is the *Shigella flexneri* IIa group (3, 4) antigen and the genetic hybrid strain expresses both *Salmonella typhi* and *Shigella flexneri* IIa group (3, 4) antigens.

6. An oral vaccine according to Claim 4 wherein the *Shigella* strain protective antigen is the *Shigella flexneri* IIa group (3, 4) and type antigens and the genetic hybrid strain expresses both *Salmonella typhi,* and *Shigella flexneri* IIa group (3, 4) and type antigens.

7. An oral vaccine according to Claim 1 further comprising a pharmaceutical diluent.

8. An oral vaccine according to Claim 1 in freeze-dried form.

9. A genetic hybrid derivative of an attenuated galactose epimeraseless mutant strain of *Salmonella typhi* and a *Shigella* strain protective antigen carried thereby.

10. A genetic hybrid derivative according to Claim 9 wherein the *Shigella* strain protective antigen is the form I antigen of *Shigella sonnei* and the genetic hybrid strain expresses both *Salmonella typhi* and *Shigella sonnei* antigens.

11. A genetic hybrid derivative according to Claim 10 wherein the form I antigen is plasmid encoded.

12. A genetic hybrid derivative according to Claim 9 wherein the *Shigella* strain protective antigen is a *Shigella flexneri* protective antigen.

13. A genetic hybrid derivative according to Claim 12 wherein the *Shigella* strain protective antigen is the *Shigella flexneri* IIa group (3, 4) antigen and the genetic hybrid strain expresses both *Salmonella typhi* and *Shigella flexneri* IIa group (3, 4) antigens.

14. A genetic hybrid derivative according to Claim 12 wherein the *Shigella* strain protective antigen is the *Shigella flexneri* IIa group (3, 4) and type antigens and the genetic hybrid strain expresses both *S. flexneri* IIa group (3, 4) and type antigens.

**Patentansprüche**

1. Eine lebende abgeschwächte orale Vakzine zur Immunisierung gegen Darmerkrankungen, enthaltend als Aktivkomponente eine wirksame Dosis eines genetischen Hybridderivats eines abgeschwächten galactose-epimerasefreien Mutantenstammes von *Salmonella typhi* und eines davon getragenen *Shigella*-Stamm-Schutzantigens oder ein Gemisch dieser genetischen Hybridderivate.

2. Orale Vakzine gemäß Anspruch 1, worin das *Shigella*-Stamm-Schutzantigen das Form I-Antigen von *Shigella sonnei* ist und der genetische Hybridstamm sowohl *Salmonella typhials* auch *Shigella sonnei*-Antigene exprimiert.

3. Orale Vakzine gemäß Anspruch 2, worin das Form I-Antigen durch ein Plasmid kodiert ist.

4. Orale Vakzine gemäß Anspruch 1, worin das *Shigella*-Stamm-Schutzantigen ein *Shigella flexneri*-Schutzantigen ist.

5. Orale Vakzine gemäß Anspruch 4, worin das *Shigella*-Stamm-Schutzantigen das *Shigella flexneri* IIa-Gruppen (3, 4)-Antigen ist und der genetische Hybridstamm sowohl *Salmonella typhi* als auch *Shigella flexneri* IIa-Gruppen (3, 4)-Antigene exprimiert.

6. Orale Vakzine gemäß Anspruch 4, worin das *Shigella*-Stamm-Schutzantigen *Shigella flexneri* IIa-Gruppen (3,4)- und Typen-Antigen ist und der genetische Hybridstamm sowohl *Salmonella typhi* als auch *Shigella flexneri* IIa-Gruppen(3,4)- und Typenantigene exprimiert.

7. Orale Vakzine gemäß Anspruch 1, die ferner ein pharmazeutisches Verdünnungsmittel enthält.

8. Orale Vakzine gemäß Anspruch 1 in gefriergetrockneter Form.

9. Genetisches Hybridderivat eines abgeschwächten galactose-epimerasefreien Mutantenstammes von *Salmonella* typhi und eines davon getragenen *Shigella*-Stamm-Schutzantigens.

10. Genetisches Hybridderivat nach Anspruch 9, worin das *Shigella*-Stamm-Schutzantigen das Form I-Antigen von *Shigella sonnei* ist und der genetische Hybridstamm sowohl *Salmonella typhi*- als auch *Shigella sonnei*-Antigene exprimiert.

11. Genetisches Hybridderivat nach Anspruch 10, worin das Form I-Antigen durch ein Plasmid kodiert ist.

12. Genetisches Hybridderivat nach Anspruch 9, worin das *Shigella*-Stamm-Schutzantigen ein *Shigella flexneri*-Schutzantigen ist.

13. Genetisches Hybridderivat nach Anspruch 12, worin das *Shigella*-Stamm-Schutzantigen das *Shigella flexneri* IIa-Gruppen(3,4)-Antigen ist und der genetische Hybridstamm sowohl *Salmonella typhi* als auch *Shigella flexneri* IIa-Gruppen(3,4)-Antigene exprimiert.

14. Genetisches Hybridderivat gemäß Anspruch 12, worin das Shigella-Stamm-Schutzantigen *Shigella flexneri* IIa-Gruppen(3,4)- und Typen-Antigen ist und der genetische Hybridstamm sowohl *Shigella flexneri* IIa-Gruppen(3,4)-Antigene als auch Typenantigene exprimiert.

**Revendications**

1. Vaccin oral atténué vivant, pour l'immunisation contre une maladie entérique, comprenant comme composant actif une dose efficace d'un dérivée hybride génétique d'une souche mutante atténuée sans galactose épimérase de Salmonella typhi et d'un antigène protecteur d'une souche Shigella porté par ce moyen ou un mélange de ces dérivés hybrides génétiques.

2. Vaccin oral selon la revendication 1, dans lequel l'antigène protecteur de la souche Shigella est l'antigène de forme I de Shigella sonnei et la souche hybride génétique exprime à la fois les antigènes de Salmonella typhi et de Shigella sonnei.

3. Vaccin oral selon la revendication 2, dans lequel l'antigène de forme I est codé par un plasmide.

4. Vaccin oral selon la revendication 1, dans lequel l'antigène protecteur de la souche Shigella est un antigène protecteur de Shigella flexneri.

5. Vaccin oral selon la revendication 4, dans lequel l'antigène protecteur de la souche Shigella est l'antigène du groupe (3, 4) de Shigella flexneri IIa et la souche hybride génétique exprime à la fois les antigènes du Salmonella typhi et du groupe (3, 4) de Shigella flexneri IIa.

6. Vaccin oral selon la revendication 4, dans lequel l'antigène protecteur de la souche Shigella est représenté par les antigènes du type et du groupe (3, 4) de Shigella flexneri IIa et la souche hybride génétique exprime à la fois les antigènes de Salmonella typhi et du type et du groupe (3, 4) de Shigella flexneri IIa.

7. Vaccin oral selon la revendication 1, comprenant de plus un diluant pharmaceutique.

8. Vaccin oral selon la revendication 1, sous une forme lyophilisée.

9. Dérivé hybride génétique d'une souche mutante atténuée sans galactose épimérase de Salmonella typhi et d'un antigène protecteur d'une souche Shigella porté par ce moyen.

10. Dérivé hybride génétique selon la revendication 9, dans lequel l'antigène protecteur de la souche Shigella est l'antigène de forme I de Shigella sonnei et la souche hybride génétique exprime à la fois les antigènes de Salmonella typhi et de Shigella sonnei.

11. Dérivé hybride génétique selon la revendication 10, dans lequel l'antigène de forme I est codé par un plasmide.

12. Dérivé hybride génétique selon la revendication 9, dans lequel l'antigène protecteur de la souche Shigella est un antigène protecteur de Shigella flexneri.

13. Dérivé hybride génétique selon la revendication 12, dans lequel l'antigène protecteur de la souche Shigella est l'antigène du groupe (3, 4) de Shigella flexneri IIa et la souche hybride génétique exprime à la fois les antigènes de Salmonella typhi et du groupe (3, 4) de Shigella flexneri IIa.

14. Dérivé hybride génétique selon la revendication 12, dans lequel l'antigène protecteur de la souche Shigella est représenté par les antigènes du type et du groupe (3, 4) de Shigella flexneri IIa et la souche hybride génétique exprime à la fois des antigènes du type et du groupe (3, 4) de Shigella flexneri IIa.

FIG. 1

O. D. 650

HOURS

FIG. 2

Ty2 + galactose

Ty 2la

5076-IC

5076-IC + galactose

Ty 2la + galactose

0 088 108